# EUROPEAN PATENT APPLICATION

(11) **EP 4 617 351 A1**
(43) Date of publication of application: **17.09.2025**
(21) Application number: 25163480.4
(22) Date of filing: 13.03.2025
(51) Int. Cl.: C12M 1/00, C12M 1/26, C12M 1/34, C12M 1/36

(54) **CELL DETACHMENT SYSTEM AND CELL DETACHMENT METHOD**

(30) Priority: 15.03.2024 JP 2024040944
(71) Applicant: CANON KABUSHIKI KAISHA, Tokyo 146-8501 (JP)
(72) Inventor: NAKAMOTO, Atsushi, Tokyo (JP); SURUGA, Eika, Tokyo (JP); YOSHIDA, Ryoichi, Tokyo (JP)
(74) Representative: WESER & Kollegen Patentanwälte PartmbB

(57) **Abstract**

A cell detachment system that detaches a cell sheet adhering to a culture container (8) from the culture container (8) includes a detachment start unit that starts detachment of the cell sheet; an information acquisition unit that acquires information regarding a detachment progressing direction of the cell sheet after start of the detachment of the cell sheet; a shaking unit (3) that shakes the culture container (8); and a setting unit that sets, on a basis of the information regarding the detachment progressing direction, the detachment progressing direction and a direction of the shaking to be substantially parallel to each other.

## Description

### BACKGROUND

### Field of the Disclosure

The present disclosure relates to a cell detachment system and a cell detachment method.

### Description of the Related Art

In the fields of regenerative medicine and cell medicine, recent years have seen attempts to culture cells in sheet forms and transplant the cultured sheet-shaped cells (cell sheets) to affected areas to repair damaged tissues, etc. When cell sheets are produced by using adherent cells, for example, cells are cultured into a sheet on a polystyrene dish, which is one example of a culture substrate, and are detached and recovered as a sheet from the culture substrate. Studies have been done on the method for efficiently and stably producing cell sheets while reducing wrinkles, tears, holes, etc., in the cell sheets.

Japanese Patent Laid-Open No. 2014-113133 discloses a cell detachment apparatus that includes a container holder for attaching a culture container to which cultured cells have adhered and a guide mechanism that guides back-and-forth movement of the container holder, in which the container holder is allowed to collide with a collision target member.

### SUMMARY OF THE DISCLOSURE

The present disclosure provides a cell detachment system that efficiently detaches a cell sheet while reducing tearing of the cell sheet. The present disclosure also provides a cell detachment method that efficiently detaches a cell sheet while reducing tearing of the cell sheet.

The present disclosure in its first aspect provides a cell detachment system as specified in claims 1 to 10.

The present disclosure in its second aspect provides a cell detachment method as specified in claim 11.

Further features of the present disclosure will become apparent from the following description of exemplary embodiments with reference to the attached drawings.

### BRIEF DESCRIPTION OF THE DRAWINGS

Fig. 1 is a schematic view of a cell detachment system according to a first embodiment.
Fig. 2 is a cross-sectional view of a tapping unit according to the first embodiment.
Fig. 3 is a schematic view of a structure that generates a local water flow according to the first embodiment.
Fig. 4 is a schematic view of a structure that starts detachment by applying ultrasonic vibrations according to the first embodiment.
Fig. 5A and Fig. 5B are each a schematic view of a detached state of the cell sheet according to the first embodiment.
Fig. 6 is a schematic view of a structure that acquires information regarding the detachment progressing direction by referencing a database according to the first embodiment.
Figs. 7A and 7B are schematic views of a uniaxial shaking mechanism according to the first embodiment.
Fig. 8 is a schematic view of a biaxial shaking mechanism according to the first embodiment.
Fig. 9 is a schematic view of a mechanism that changes the tapping direction according to the first embodiment.
Fig. 10 is a schematic view of a mechanism that rotates a culture container according to the first embodiment.
Fig. 11 is a flowchart schematically illustrating processes that do not include direction changes according to the first embodiment.
Fig. 12 is a flowchart schematically illustrating processes that include direction changes according to the first embodiment.
Fig. 13 is a cross-sectional view of a mechanism that applies vertical vibrations according to the first embodiment.

### DESCRIPTION OF THE EMBODIMENTS

When a cell sheet is being detached from a culture container that retains a liquid and has the cell sheet adhered thereto and when the direction in which the culture container is allowed to undergo back-and-forth movement is the same as the direction in which the impact is applied to the culture container, there has been instances where the cell sheet is prone to tearing. Under conditions employed for efficiently detaching a cell sheet in a shorter period of time, there has also been instances where the cell sheet is prone to tearing.

The present disclosure will now be described in detail through embodiments.

The inventors of the present disclosure have investigated an efficient method for detaching a cell sheet. As a result, it has been found that detachment of a cell sheet can be promoted while reducing tearing of the cell sheet when a flow of a liquid inside the culture container is generated in a specified direction with respect to the cell sheet that has started to detach from one part of an outer edge.

Specifically, it has been elucidated that a cell sheet can be efficiently detached while reducing tearing of the cell sheet by shaking the culture container in a direction substantially parallel to the direction in which the cell sheet that has started to detach is detaching. Here, substantially parallel means that at least one of the angles formed by the two directions is 45° or less, 30° or less, or 15° or less.

The reason why the cell sheet can be detached while reducing tearing of the cell sheet by adjusting the angle formed between the direction of detachment and the direction of shaking is presumably as follows. The flow of the liquid generated by shaking the culture container enters the gap between the culture container and the cell sheet that has partly detached, and pushes up the cell sheet in a direction of further detaching the cell sheet. During this process, presumably since the water flow acts along the detachment direction with respect to the partly detached cell sheet, excessive concentration of force on the cell sheet is reduced, and detachment is promoted.

A cell detachment system according to one embodiment of the present disclosure will now be described.

### First embodiment

### Gist of cell detachment system

A cell detachment system according to a first embodiment detaches a cell sheet adhering to a culture surface of a culture container from the culture surface through the following processes. The cell detachment system of this embodiment performs a detachment start process of starting detachment through a detachment start unit; and an information acquisition process of acquiring information regarding a direction in which detachment of the cell sheet is progressing through an information acquisition unit after starting the detachment. The cell detachment system of this embodiment further performs a shaking process of shaking a culture container by using a shaking unit and a setting process of setting a direction in which the detachment is progressing and a direction of shaking to be substantially parallel to each other through a setting unit. Example of the cell detachment start step includes any one of a step of tapping a culture container culturing the cell sheet, a step of causing a water flow to locally contact the cell sheet, and a step of application of vibrations in the ultrasonic band (ultrasonic vibrations) to a culture container culturing the cell sheet.

### Cell sheet

A cell sheet in this embodiment refers to a film in which cells are interconnected to form a sheet. The cells constituting the cell sheet are not particularly limited as long as the cells are able to form a cell sheet. Examples of the cells include adherend cells such as adherend body cells.

Examples of the body cells include myoblasts (for example, skeletal myoblasts), muscle satellite cells, and mesenchymal stem cells (for example, those derived from bone marrow, adipose tissue, peripheral blood, skin, hair roots, muscle tissue, endometrium, placenta, umbilical cord blood, etc.). Other examples include cardiomyocytes, fibroblasts, tissue stem cells such as cardiac stem cells, embryonic stem cells, pluripotent stem cells such as iPS cells, synovial cells, chondrocytes, and epithelial cells (for example, oral mucosa epithelial cells, retinal pigment epithelial cells, nasal mucosa epithelial cells, etc.)

Yet other examples include endothelial cells (for example, vascular endothelial cells, etc.), liver cells (for example, liver parenchymal cells, etc.), pancreatic cells (for example, pancreatic islet cells, etc.), kidney cells, adrenal cells, periodontal ligament cells, gingival cells, periosteal cells, and skin cells.

Moreover, the body cells may be those differentiated from iPS cells (iPS cell-derived cells). Examples of the iPS cell-derived cells include cardiomyocytes, fibroblasts, myoblasts, epithelial cells, endothelial cells, hepatic cells, pancreatic cells, kidney cells, adrenal cells, periodontal ligament cells, gingival cells, periosteal cells, skin cells, synovial cells, chondrocytes, etc., derived from iPS cells.

### Culture container

The culture container of this embodiment is not particularly limited as long as the culture container is a cell-adhering culture container. For example, the culture container is any one of flasks, tissue culture flasks, dishes, Petri dishes, tissue culture dishes, multi-dishes, microplates, multi-well plates, multi-plates, culture bags, and bottles.

The material for the culture container of this embodiment may be any material that is chemically stable and that can culture desired cells. Examples of the material include polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meth)acrylic acid, poly(meth)acrylic acid derivatives, polyacrylonitrile, poly(meth)acrylamide, poly(meth)acrylamide derivatives, polysulfone, cellulose, cellulose derivatives, polysilicone, polymethylpentene, glass, and metal. Among these, polystyrene may be used from the viewpoint of stability.

Alternatively, a temperature responsive container that has a culture surface undergoing changes in hydrophilic properties by temperature may be used as the culture container of this embodiment.

### Cell remover

A cell remover in this description refers to a solution that is retained in the culture container when cells are detached by the cell detachment method according to the present disclosure. Here, the cell remover in this description does not have to contain a component that promotes detachment of the cell sheet. For example, from the viewpoint of protecting the state of the cell surfaces, a cell remover substantially free of proteolytic enzymes may be used.

Here, "substantially free of' means that the content is equal to or lower than 0.0005 mass%.

The pH of the cell remover in this embodiment can be in the neutral or acidic range.

The neutral range is suitable for cell culture and the survival rate of the cells can be stably maintained high. The pH can be adjusted, as appropriate, by using hydrochloric acid, sodium hydroxide, etc. In order to stabilize the pH, various buffer solutions may also be used.

Any buffer solution can be used without limitations in this embodiment as long as the neutral range can be maintained. Examples thereof include Tris buffer solutions such as Tris-HCl buffer solutions, phosphate buffer solutions, HEPES buffer solutions, citrate-phosphate buffer solutions, glycylglycine-sodium hydroxide buffer solutions, Britton-Robinson buffer solutions, and GTA buffer solutions. Among these, phosphate buffer solutions close to the in-vivo environment can be used, such as a phosphate buffered saline (PBS) obtained by adjusting a phosphate buffer solution adjusted to be isotonic with the intracellular fluid.

The viscosity of the cell remover in this embodiment can be 1.80 mPa·s or less. This is because flowing of the remover generated by ultrasonic vibrations is not obstructed, and the detachment efficiency can be maintained high. The viscosity of the cell remover can be adjusted, as appropriate, by adding a polymer, a saccharide, etc.

The cell remover of this embodiment may contain a proteolytic enzyme, but the amount of the proteolytic enzyme relative to the total mass of the cell remover can be 0.0005 mass% or less or zero. This is because a proteolytic enzyme possibly degrades the quality of the cells although the detachment efficiency can be increased as the proteolytic enzyme breaks down some part of the cells.

A proteolytic enzyme in this embodiment is an enzyme that breaks down some part of cells to facilitate detachment of the cells from the substrate. Examples thereof include trypsin, accutase, collagenase, natural proteases, chymotrypsin, elastase, papain, pronase, and recombinants thereof.

The cell remover in this embodiment may be a medium but is not particularly limited. A solution that contains a metal ion chelating agent (hereinafter may also be referred to as a chelating agent) may also be used. This is because using a cell remover containing a chelating agent can more effectively detach the cells by ultrasonic vibrations.

The chelating agent in this embodiment is not particularly limited. Examples of the chelating agent include ethylenediaminetetraacetic acid (hereinafter may also be referred to as EDTA), ethylenediamine, ethylenediaminetetramethylenephosphonic acid, glycol ether diaminetetraacetic acid, nitrilotriacetic acid, diethylenetriaminepentaacetic acid, iminodiacetic acid, dihydroxyethylglycine, dicarboxymethylglutamic acid, ethylenediaminedisuccinic acid, etidronic acid, citric acid, gluconic acid, and phosphonobutanetriacetic acid.

Among these, the chelating agent can form a chelate with a divalent cation, or can form a chelate with Ca2+ and Mg2+, and the chelating agent can be ethylenediaminetetraacetic acid. When ethylenediaminetetraacetic acid is used as the chelating agent, the pH of the cell remover may be 7.0 or more and 8.0 or less. This is because this level is rather high in the neutral range where the cell survival rate can be maintained high, and thus the chelating ability of ethylenediaminetetraacetic acid can be further enhanced and the detachment efficiency can be further enhanced.

The chelating agents can be used alone or in combination. The chelating agent content can be 0.01 mM or more and 5.0 mM or less. Within this range, the chelating effect can be securely obtained, and the decrease in activity caused by the presence of excessive chelating agents can also be reduced.

The cell remover of this embodiment may contain a hydrophilic polymer including a polyalkylene glycol structure. Polyalkylene glycol can increase the survival rate of the cells in the cell detachment method using ultrasonic waves. An example of the hydrophilic polymer including a polyalkylene glycol structure is polyethylene glycol. The hydrophilic polymer can have a peak molecular weight Mp of 800 or more and 50000 or less or can have a peak molecular weight Mp of 1200 or more and 20000 or less as measured by gel permeation chromatography. This is because the influence of the polymer on the cells is small, and the medium thickening effect caused by the polymer can be reduced.

The type of the medium may be any, and examples thereof include a Dulbecco's Modified Eagles's Medium (DMEM), Ham's Nutrient Mixture F12, a DMEM/F12 medium, a McCoy's 5A medium, an Eagles's Minimum Essential Medium (EMEM), α Modified Eagles's Minimum Essential Medium (αMEM), a Minimum Essential Medium (MEM), an RPMI-1640 medium, an Iscove's Modified Dulbecco's Medium (IMDM), an MCDB 131 medium, a William's E medium, an IPL-41 medium, a Fischer's medium, StemSpan H3000, StemSpan SFEM, Stemline II, Endothelial Cell Growth Medium 2 Kit, Mesenchymal Stem Cell Growth Medium 2, MSCGM Bullet Kit, mTeSR1, an mTeSR2 medium, Repro FF, Repro FF2, a NutriStem medium, and an MF-Medium Mesenchymal Stem Cell Growth Medium.

A medium that is suitable for culturing each cell type may be used among these.

A serum or an antibiotic may be added the aforementioned medium. Examples of the serum include fetal bovine serum (FBS), calf serum, adult bovine serum, horse serum, sheep serum, goat serum, pig serum, chicken serum, rabbit serum, and human serum, and in general, FBS is frequently used for its high availability. Alternatively, a serum-free medium that does not contain any of untreated or unpurified serums but contains purified blood-derived components or animal tissue-derived components (growth factors, etc.) may be used.

Examples of the antibiotic added to the medium include penicillin, streptomycin, ampicillin, carbenicillin, tetracycline, bleomycin, actinomycin, kanamycin, actinomycin D, and amphotericin B.

### Cell sheet culture conditions

The cell sheet culture conditions can be selected as appropriate for the cells to be cultured. Typically, an appropriate medium is added to a dish, about 1.0 × 10¹ to 5.0 × 10⁴ cells/cm² of cells are seeded therein, and culture is carried out in an environment at a temperature of 37°C and a CO₂ concentration of 5%. Here, culture may be carried out until the cell-occupied area ratio in the substrate is about 100%, in other words, until a confluent state is reached.

### Cell sheet detachment start unit

Examples of the cell sheet detachment start unit include tapping the culture container culturing the cell sheet, causing a water flow to locally contact the cell sheet, and application of vibrations in the ultrasonic band (ultrasonic vibrations) to the culture container. These may be used alone or in combination.

### Tapping unit

Start of detachment can be urged by performing a tapping process of applying, by using a tapping unit as the detachment start unit, an impulsive force to the culture container in which the cell sheet is cultured.

The tapping process in this embodiment includes a process of applying a repulsive force to the culture substrate in order to detach at least part of the cells adhering to the culture surface of the culture substrate from the culture surface. The tapping process is, for example, a process of performing at least one of a process of applying a repulsive force to the culture substrate by tapping the culture substrate itself, and a process of applying a repulsive force to the culture substrate by moving the culture substrate and causing the culture substrate to collide with a member that constitutes a cell detachment apparatus.

The tapping process in this embodiment may be periodic or non-periodic, and the magnitude of the repulsive force applied to the culture substrate in the tapping process may be set appropriately. The tapping process in this embodiment may be continuously performed from the start to the completion of the cell detachment, may be terminated before completion, or may be performed intermittently.

The tapping unit in this embodiment may be any feature that can carry out the aforementioned tapping process. The tapping unit includes, for example, an object to be collided against the culture substrate and a moving unit that moves this object to collide against the culture substrate, or includes a moving unit that moves the culture substrate to collide with a member constituting the cell detachment apparatus. The object may have a mass that can apply an appropriate repulsive force to the culture substrate without tearing the culture substrate.

Examples of the shape of the object include a rod shape, a hammer shape, and a spherical shape. Examples of the moving unit include motors and solenoids that can generate a moving force that moves the object or the culture substrate through electrification. Here, the object or the culture substrate may be moved by using only the motors, solenoids, etc., or the motors and solenoids may be used in combination with a member, such as a spring member, that can store energy.

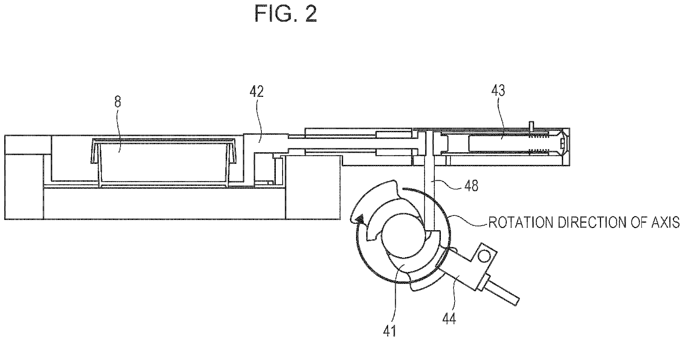

Fig. 2 is a cross-sectional view of one example of the tapping unit of this embodiment. A cam 41 is connected to a motor, and rotates in the rotation direction of the axis when driven by the motor. A hammer 42 is in contact with the cam 41 at a shaft 48, and is urged in a direction of tapping a culture container 8 by a spring 43. After the spring 43 is compressed by the rotation of the cam 41, the hammer 42 moves in such a direction that the profile of the cam 41 taps the culture container 8. The frequency of tapping can be determined by the rotation rate of the motor, and can be detected with a photocoupler 44.

### Liquid discharging unit

A liquid discharging unit that discharges a liquid can be used as the detachment start unit. The water flow generated by the discharge of the liquid is brought into contact with the cell sheet to promote detachment of the cell sheet. The liquid discharging unit is, for example, a pipette. The unit for generating the water flow is not limited to this, and a structure such as a nozzle or a channel can be employed. The water flow generated by the discharge of the liquid can be brought into contact with an edge portion (outer edge portion) of the cell sheet from the viewpoint of efficiently detaching the cell sheet.

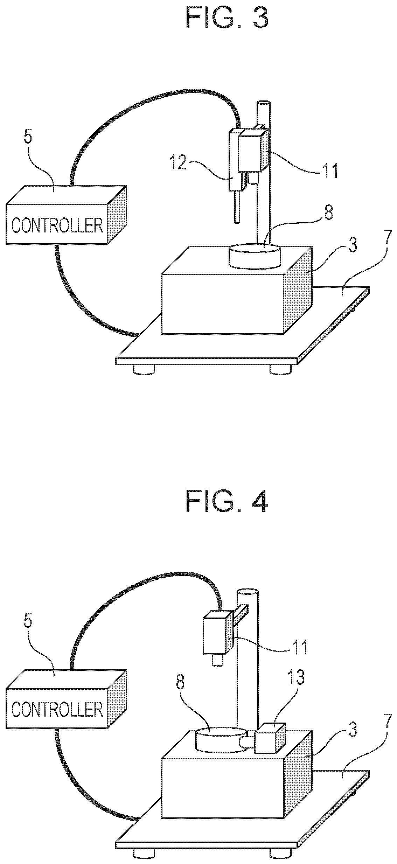

Fig. 3 is a schematic diagram of the structure of a liquid discharging unit of this embodiment. An electric pipette 12 is controlled by a controller 5, and a local water flow can be brought into contact with an edge portion of the culture container 8. The medium discharged from the electric pipette 12 generates a water flow directed from a bottom end portion to a bottom center portion of the culture container 8. This water flow can apply a force that starts detachment of the edge portion of the cell sheet.

### Starting detachment by ultrasonic vibrations

An ultrasonic wave generating unit can be used as another example of the detachment start unit. The ultrasonic wave generating unit starts detachment of the cell sheet adhering to the culture container by applying vibrations in the ultrasonic band to the culture container. Fig. 4 is a schematic diagram of the structure that starts detachment by ultrasonic vibrations in this embodiment. An ultrasonic wave generating unit 13 is controlled by a controller 5, and can apply ultrasonic waves to the culture container 8. The ultrasonic vibrations can apply a force that starts detachment from an outer edge portion of the cell sheet in the culture container 8.

Examples of the vibrations in the ultrasonic band used in the detachment start unit are vibrations having frequencies of 10 kHz or more and 1 MHz or less. The ultrasonic wave generating unit can be any unit that can apply vibrations in the ultrasonic band to the cells. One example is where an ultrasonic oscillator such as lead zirconium titanate (PZT) is used as a vibrating body.

The ultrasonic vibrating body may be any body that can generate ultrasonic waves, and an example thereof is a piezoelectric body and a vibrating plate bonded together. When the piezoelectric body has a circular shape, the vibrating plate may be glass, SUS, or quartz. When the vibrating plate is glass, SUS, or quartz, large amplitudes can be output at a relatively high driving frequency (vibration frequency) in the ultrasonic wave region without breaking the ultrasonic vibrating body.

In the case of a ring-shaped piezoelectric body, the outer diameter of the vibrating plate may be equal to the outer diameter of the piezoelectric body. The thickness of the vibrating plate may be such that, when the vibrating plate and the piezoelectric body are bonded together and undergo deflection vibrations, the midpoint of the deflection in the thickness direction, that is, the neutral plane that does not undergo either tension or contraction during deflection, may be on the vibrating plate side since the strain in the piezoelectric body can be highly efficiently used in the deflection.

Furthermore, a commercially available Langevin oscillator or rectangular oscillator can also be used as the ultrasonic vibrating body of the present disclosure. An example of the Langevin oscillator is an oscillator in which a piezoelectric body is interposed between two metal blocks and clamped together to form an integrated structure with bolts or the like.

The operation of the detachment start unit may be performed periodically or may be continued until the detachment of the cell sheet is complete.

### Acquisition of information regarding detachment progressing direction by observation unit

An example of a unit that acquires information regarding the direction in which the detachment progresses is an observation unit, such as a camera, that observes the cell sheet and acquires the observation results as image information. Alternatively, a measurement unit such as a distance measurement sensor that acquires the adhering state of the cell sheet onto the inner surface of the culture container as numerical information.

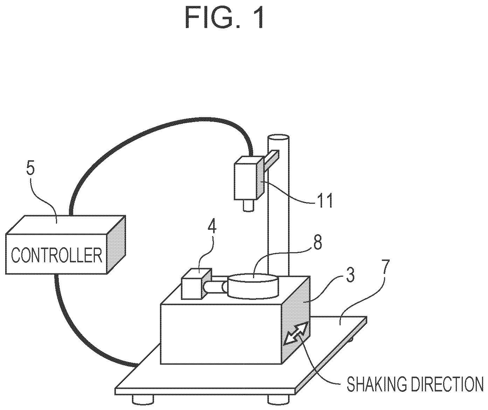

Fig. 1 a schematic view of a cell detachment system according to this embodiment that includes an observation unit 11. The observation unit 11 is, for example, a commercially available camera or microscope, is controlled by a controller 5, and enables observation of the state of the cell sheet in the culture container 8.

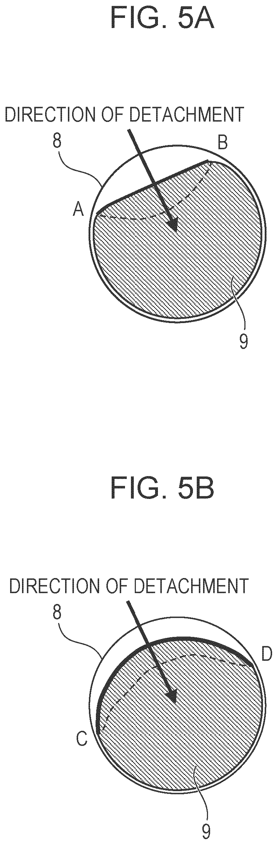

Figs. 5A and 5B each illustrate the detached state of the cell sheet 9, and a detachment boundary line AB indicates the adherence-detachment boundary of the cell sheet 9. For example, in the state illustrated in Fig. 5A, the detachment direction can be determined to be a direction perpendicular to the detachment boundary line AB or a direction that connects the midpoint of the detachment boundary line AB and the center of gravity of the cell sheet 9, for example. In addition, in the case where a detachment boundary curve CD forms an arc as illustrated in Fig. 5B, the detachment direction can be determined to be a direction normal to the midpoint of the detachment boundary curve CD or a direction that connects the midpoint of the detachment boundary curve CD and the center of gravity of the cell sheet 9, for example. In this embodiment, a direction normal to the midpoint of the detachment boundary curve CD is defined as the detachment direction.

### Acquisition of information regarding detachment progressing direction through database

In other instances, the information acquisition unit may reference a database in which information regarding the culture container and the detachment progressing direction for each culture container are stored in association with each other. The database in which the detachment progressing direction and the information regarding the culture container are preliminarily associated with each other may be stored in a memory region of the detachment system or in a server connected via a network. The information regarding the culture container is, for example, at least one of the size, shape, manufacturer, and preassigned number of the culture container.

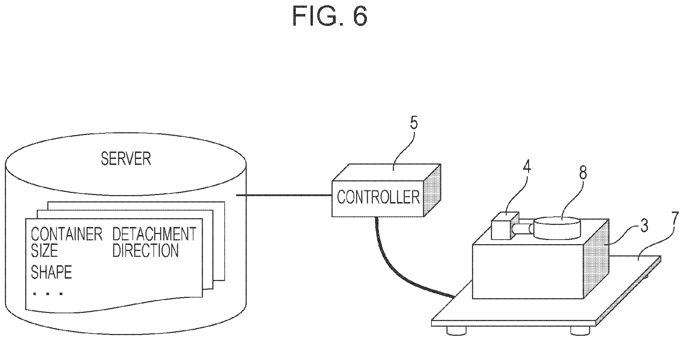

Fig. 6 is a schematic view of a structure that acquires information regarding the detachment progressing direction by referencing the database in this embodiment. The technique of referencing the database enables acquisition of information regarding the detachment progressing direction without observation of the cell sheet, etc., and thus the number of structures and the size of the system can be reduced.

The information regarding the detachment progressing direction may be acquired multiple times during detachment or may be additionally acquired even before start of the detachment. In order to acquire a more accurate detachment direction, information acquisition may be performed multiple times according to the progress of the detachment, and information may be updated in response to the acquired information of the detachment progressing direction.

### Shaking culture container

In this embodiment, shaking may involve a feature of generating a flow in a liquid inside the culture container by applying an acceleration containing a direction component parallel to the culture surface of the culture container in which the cell sheet is cultured. For example, a feature that generates rotation movement by a motor and perform uniaxial back-and-forth movement by using slide rails or the like can be used. Alternatively, a feature of applying an acceleration containing a direction component parallel to the culture surface by accelerating the culture container with a linear motor or the like can also be employed. As long as an acceleration containing a direction component parallel to the culture surface of the culture container can be added by shaking, the axis of shaking itself does not have to be parallel to the culture surface.

In this embodiment, the amplitude of shaking can be 0.1 mm or more and 300 mm or less or can be 0.1 mm or more and 150 mm or less. For the back-and-forth movement, the frequency can be 0.1 Hz or more and 20 Hz or less or can be 0.1 Hz or more and 10 Hz or less.

### Uniaxial shaking mechanism

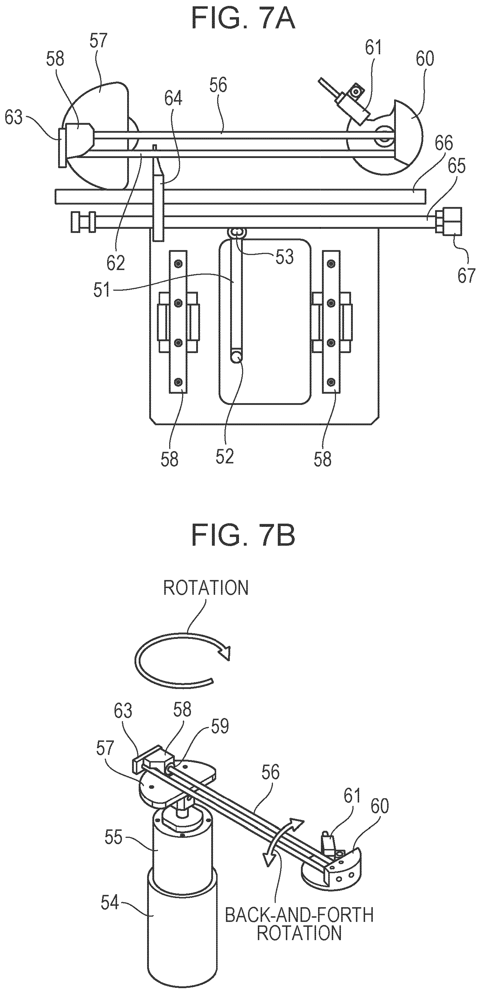

Figs. 7A and 7B illustrate a uniaxial shaking mechanism according to this embodiment. Guide rails 50 are fastened to a base plate 7 illustrated in Fig. 1 so as to shake the culture substrate 8 back and forth. A base-side spring post 52 to which a pressure spring 51 is connected is fixed to the base plate 7, and the pressure spring 51 is connected to the base-side spring post 52. The opposite end portion of the pressure spring 51 is fixed to a shaken-side spring post 53 provided in a shaken portion. The pressure spring 51 can be selected as necessary and has a detachable structure.

Shaking occurs as rotations of a shake motor 54 are transmitted to a shake rod 56 through a gear 55. A rotation disk 57 is fixed to the rotation axis of the shake motor 54 and rotates as illustrated in the drawing.

The rotation direction may be reversed. A linear bush holder 58 is rotatably installed at a position offset from the rotation axis, a linear bush 59 is built therein, and the shake rod 56 is built therein to be linearly movable. One end of the shake rod 56 is fixed to a rotatable shake fulcrum shaft 60. This translates movement into back-and-forth movement centered on the shake fulcrum shaft 60. The frequency of the back-and-forth movement is detected with a rotation photocoupler 61.

A connecting rod 62 is fastened to the shake rod 56 with a connecting rod fixing tool 63 so as to be parallel to the shake rod 56, and one end is fixed to the shake fulcrum shaft 60. A shake slide piece 64 equipped with the base plate 7 contacts the connecting rod 62 moving back and forth to generate back-and-forth shaking movement. The shake slide piece 64 is supported by a piece slide shaft 66 parallel to a shake adjust screw rod 65, and the shaking strokes can be freely adjusted by rotating the shake adjust screw rod 65. A stroke tab 67 is installed at an end of the shake adjust screw rod 65, and can be manually rotated to perform adjustment.

### Biaxial shaking mechanism

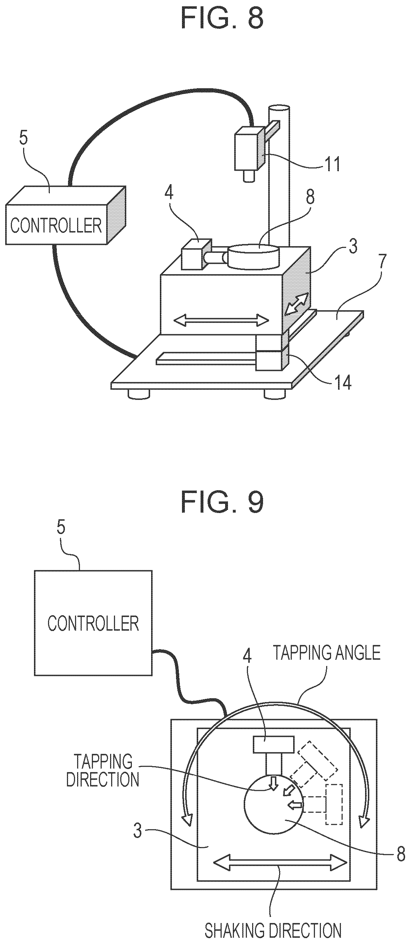

Fig. 8 is a schematic view of a biaxial shaking mechanism according to this embodiment. Linear rails 14 orthogonal to each other are biaxially arranged so that the shaking direction can be freely changed. The linear rails 14 are controlled by the controller 5, and the detachment progressing direction and the shaking direction can be controlled to be substantially parallel to each other.

### Setting unit that sets detachment progressing direction and shaking direction to be substantially parallel to each other

In this embodiment, the setting unit that sets the detachment progressing direction and the shaking direction to be substantially parallel to each other can change the direction in which the detachment start unit is applied according to the detachment progressing direction. In addition, the shaking direction may be changed in accordance with the detachment progressing direction and/or the orientation of the culture container may be changed in accordance with the detachment progressing direction. These two changes may be separately implemented or two or more such changes may be implemented together.

### Changing tapping direction

Fig. 9 is a schematic view of a mechanism that changes the tapping direction in this embodiment. A tapping unit 4 is configured such that the angle at which the culture container 8 is tapped can be freely changed, and the tapping direction can be freely changed due to this structure. When the tapping unit 4 is actuated while the tapping direction of the tapping unit 4 is changed on the basis of the tapping direction set by the setting unit, the shaking direction and the detachment progressing direction can be made substantially parallel to each other.

### Changing shaking direction

In the example illustrated in Fig. 8, the shaking direction is controlled biaxially, and the shaking direction can be changed to any angle within the operation plane. By changing the shaking direction on the basis of the shaking direction set by the setting unit, the shaking direction and the detachment progressing direction can be made substantially parallel to each other.

### Changing orientation of culture container

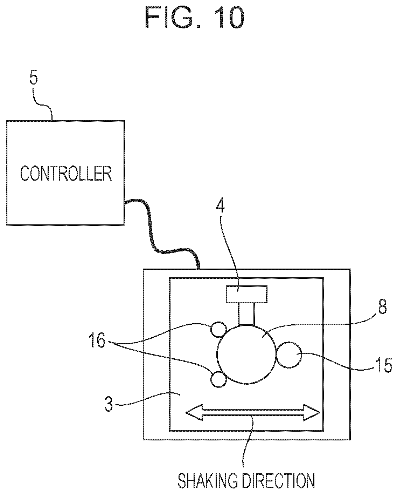

By rotating the culture container 8 after the cell sheet has started to detach from the culture container 8, the shaking direction and the detachment progressing direction can be made substantially parallel to each other. Fig. 10 is a schematic view of a mechanism that rotates the culture container 8 in this embodiment. A drive roller 15 and a roller 16 are in contact with the culture container 8, and the culture container 8 is rotated by the drive roller 15 connected to a motor. The roller 16 is driven and rotated along with the rotation of the culture container 8. The drive roller 15 has its rotation angle controlled by the controller 5 and can make the shaking direction and the detachment progressing direction substantially parallel to each other.

### Operation of cell detachment system

The operation of the cell detachment system according to this embodiment will now be described by using an example of using a tapping unit as the detachment start unit; however, note that the structures and features herein are merely examples and do not limit the scope of the present disclosure. Instead of the tapping unit, at least one of local water flow generation into the culture container by using a liquid discharging unit and application of vibrations in the ultrasonic band (ultrasonic vibrations) to the culture container can also be used.

Fig. 1 a schematic view of one example of the structure of the cell detachment system according to this embodiment. The tapping unit 4 serves as the detachment start unit and taps the culture container 8. The observation unit 11 serves as a unit that acquires information regarding the detachment progressing direction and observes the state of the cell sheet cultured within the culture container 8. The culture container 8 in which the cell sheet is cultured is set on the shaking unit 3, and the cell sheet can be detached by driving the tapping unit 4, the observation unit 11, and the shaking unit 3 controlled by the controller 5. One example of the operation is to sequentially perform the following processes in this order: tapping the culture container 8 with the tapping unit 4, detecting the detachment through the observation unit 11, and shaking the culture container 8 by the shaking unit 3 in a direction substantially parallel to the detachment direction.

It is important that the detachment direction and the shaking direction be substantially parallel to each other, and the operation timing is not particularly limited. For example, after driving the cell detachment system, the shaking process, the observation process, and the tapping process may be started simultaneously, or the direction of shaking may be changed at the stage of detecting the detachment direction by the observation process.

The order in which the tapping, shaking, and vertical vibrations such as ultrasonic vibrations are started may be any. The adhering force of the cells can be weakened while applying a shear force, and thus the cells can be more effectively detached when the tapping process and the shaking process are performed during or after application of vertical vibrations. In order to determine whether to transition to each process, the information regarding the cells adhering to the substrate may be measured and used.

The cell detachment system according to this embodiment may feature performing other processes in addition to the aforementioned processes. Examples of the other processes include a process of substituting a cell remover, a process of rinsing the cells with the cell remover, and a process of diluting the remover and homogenizing the solution inside the culture container.

The aforementioned processes may be repeated at particular periods or irregular periods. The timing may be made uniform or may be adjusted for each process. The ratio of the total time for which the tapping process and the shaking process are performed to the total time of the processes of external stimuli, such as ultrasonic waves, can be 0.01 or more and 100 or less.

### Process flow 1 by cell detachment system

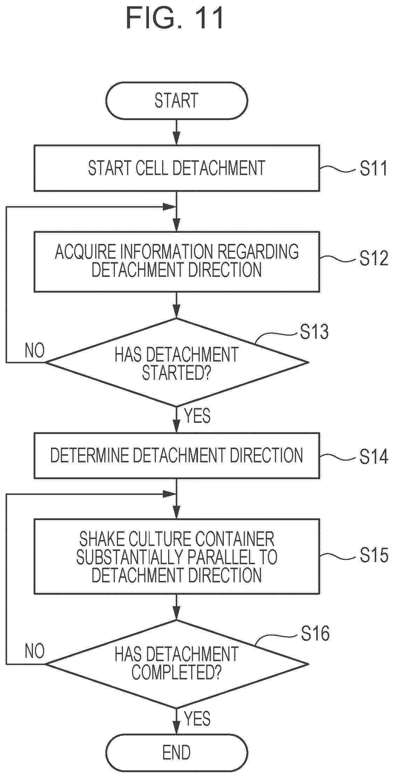

Fig. 11 is a flowchart schematically illustrating the processes performed by the cell detachment system according to this embodiment. In the process of step S11, detachment of the cells is started. In the process of step S12, information regarding the detachment direction of the cell sheet in the culture container 8 is acquired.

The information regarding the detachment direction of the cell sheet is stored in a memory region in the controller 5. In the process of step S13, on the basis of the information regarding the detachment direction of the cell sheet, whether the cell sheet has started to detach from the culture container 8 is determined.

The controller 5 may be constituted by a module executed by a CPU or an MPU, or may be constituted by, for example, a circuit that achieves particular functions, such as an ASIC. It should be noted that the present disclosure may be realized through a cell detachment system connected to multiple devices or calculation devices, and includes in its scope a cell detachment apparatus constituted by a single apparatus that realizes the same functions.

When the detachment has not started, the process transitions to the process of step S12, and step S12 and step S13 are repeated until detachment starts. When the detachment has started in the process of step S13, the process transitions to the process of step S14. In the process of step S14, on the basis of the information regarding the detachment direction of the cell sheet, the detachment direction is determined. In the process of step S15, the culture container 8 is shaken in a direction substantially parallel to the detachment direction obtained in the process of step S14.

In the process of step S16, whether the cell sheet has completely detached from the culture container 8 is determined. The state where the cell sheet has floated upward from the culture surface of the culture container 8 is determined as the completion of the detachment. When the detachment is incomplete, the process transitions to the process of step S15, and step S15 and step S16 are repeated until detachment is complete. In the process of step S16, completion of detachment ends driving of the system.

### Process flow 2 by cell detachment system

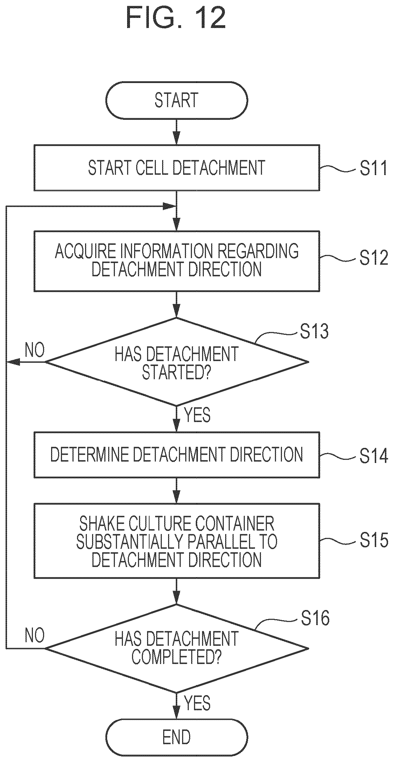

Fig. 12 is a flowchart schematically illustrating the processes performed by the cell detachment system according to this embodiment. In the process of step S11, detachment of the cells is started. In the process of step S12, information regarding the detachment direction of the cell sheet is acquired. The acquired information regarding the detachment direction of the cell sheet is stored in a memory region in the controller 5. The memory region may be configured by using any desired memory or a storage medium such as an optical disk. In the process of step S13, on the basis of the information regarding the detachment direction of the cell sheet, whether the cell sheet has started to detach from the culture container 8 is determined.

When the detachment has not started, the process transitions to the process of step S12, and step S12 and step S13 are repeated until detachment starts. When the detachment has started in the process of step S13, the process transitions to the process of step S14. In the process of step S14, on the basis of the information regarding the detachment direction of the cell sheet, the detachment direction is determined. In the process of step S15, the culture container 8 is shaken in a direction substantially parallel to the detachment direction obtained in the process of step S14.

In the process of step S16, whether the cell sheet has completely detached from the culture container 8 is determined. The state where the cell sheet has floated upward from the culture surface of the culture container 8 is determined as the completion of the detachment. When the detachment is incomplete, the process transitions to the process of step S12, and the state of the cell sheet is acquired. Through the determination in the process of step S13, the detachment direction is again determined in the process of step S14. When the detachment direction has shifted, the process of step S15 shakes the culture container 8 in a direction substantially parallel to the shifted detachment direction. The processes from step S12 to step S16 are repeated until the detachment is complete. In the process of step S16, completion of detachment ends driving of the system.

### Additional vertical vibrations by cell detachment system

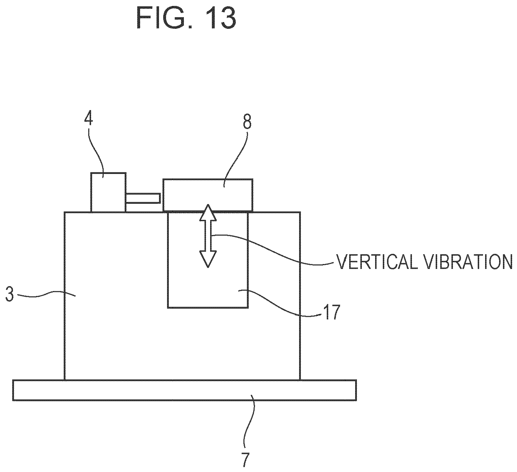

Fig. 13 is a cross sectional view of a structure in which a mechanism that applies vertical vibrations to the culture container 8 is added to the cell detachment system of this embodiment. An ultrasonic element 17 is disposed below the culture container 8 to vertically apply ultrasonic vibrations to the culture container 8. The ultrasonic element 17 is controlled by the controller 5, and the drive timing can be freely controlled.

An appropriate one of the aforementioned process flow 1 and process flow 2 is selected as the cell sheet detachment method. Alternatively, the additional vertical vibrations may be performed in addition to the process flow 1 or the process flow 2.

### Program implementing cell processing method

Furthermore, the present disclosure is also implemented by executing following processes. That is, the present disclosure may be implemented through providing software (a program) that implements one or more functions of the various embodiments described above to the system or apparatus via a network or a storage medium. The present disclosure can be implemented also by a computer (or a CPU, MPU, etc.) of the system or apparatus reading out and executing the program. The computer includes one or multiple processors or circuits, and can contain a network of multiple isolated computers or multiple isolated processors or circuits for reading out and executing the computer-executable commands.

Here, the processors or circuits may include central processing units (CPUs), micro processing units (MPUs), and graphics processing units (GPUs). Furthermore, the processors or circuits can include application specific integrated circuits (ASICs) or field programmable gate ways (FPGAs). In addition, the processors or circuits can include digital signal processors (DSPs), data flow processors (DFPs), or neural processing units (NPUs).

### Examples and Comparative Examples

The present disclosure will now be described in further detail through examples and comparative examples; however, the present disclosure is not limited in any way by the examples below without departing from the gist of the present disclosure.

### Culture of A549 cells on substrate

A549 cells, which are human lung epithelial adenocarcinoma cells, were seeded in a Φ35 temperature responsive dish (UpCell (registered trademark) produced by CellSeed Inc.) at a density of 10,000 cells/cm² and cultured in an environment of 37°C and a CO₂ concentration of 5%. The medium used was DMEM (produced by Thermo Fisher Scientific Inc.) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 6 days, and the state of the cells was observed with a phase contrast microscope to confirm cell adherence and growth. The cell-occupied area ratio of the dish was about 100%.

### Culture of C2C12 cells on substrate

C2C12 cells, which are mouse myoblast cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was a DMEM/F12 medium (produced by Thermo Fisher Scientific Inc.) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 2 days, and the state of the cells was observed with a phase contrast microscope to confirm cell adherence and growth. The cell-occupied area ratio of the dish was about 100%.

### Culture of MDCK cells on substrate

MDCK cells, which are Madin-Darby canine kidney epithelial cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was an Eagle's MEM medium (produced by FUJIFILM Wako Pure Chemical Corporation) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 8 days, and the state of the cells was observed with a phase contrast microscope to confirm cell adherence and growth. The cell-occupied area ratio of the dish was about 100%.

### Culture of HEK293 cells on substrate

HEK293 cells, which are human embryonic kidney cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was an Eagle's MEM medium (produced by FUJIFILM Wako Pure Chemical Corporation) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 9 days, and the state of the cells was observed with a phase contrast microscope to confirm cell adherence and growth. The cell-occupied area ratio of the dish was about 100%.

### Culture of BAEC cells on substrate

BAEC cells, which are bovine aorticendothelial cells, were seeded in a Φ35 polystyrene dish (produced by Corning Incorporated ) at a density of 20,000 cells/cm² and cultured in an environment of 37°C and a CO₂ concentration of 5%. The medium used was DMEM (produced by Thermo Fisher Scientific Inc.) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 7 days, and the state of the cells was observed with a phase contrast microscope to confirm cell adherence and growth. The cell-occupied area ratio of the dish was about 100%.

### Culture of hMSC cells on substrate

hMSC cells, which are human mesenchymal stem cells, were seeded in a Φ35 temperature responsive dish (UpCell (registered trademark) produced by CellSeed Inc.) at a density of 30,000 cells/cm² and cultured in an environment of 37°C and a CO₂ concentration of 5%. The medium used was an Mesenchymal Stem Cell Growth Medium 2 (produced by PromoCell GmbH) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 8 days, and the state of the cells was observed with a phase contrast microscope to confirm cell adherence and growth. The cell-occupied area ratio of the dish was about 100%.

### Culture of HUVEC cells on substrate

HUVEC cells, which are human umbilical vein endothelial cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was Endothelial Cell Growth Medium 2 Kit(produced by PromoCell GmbH) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 7 days, and the state of the cells was observed with a phase contrast microscope to confirm cell adherence and growth. The cell-occupied area ratio of the dish was about 100%.

### Detachment start unit 1: tapping of culture container

The culture container 8 was tapped by using the tapping unit illustrated in Fig. 2 as the detachment start unit 1 at a frequency of 5 Hz.

### Detachment start unit 2: local water flow

By using the liquid discharging unit illustrated in Fig. 3 as the detachment start unit 2, 1 mL of a medium was discharged in one second from the end portion of the bottom of the culture container 8 toward the center portion of the bottom.

### Detachment start unit 3: ultrasonic vibrations

By using an ultrasonic wave generating unit illustrated in Fig. 4 as the detachment start unit 3, an AC voltage having a voltage of 50 V and a frequency of 30 kHz was applied to a Langevin oscillator by the controller 5 to apply ultrasonic vibrations to the culture container.

### Shaking unit 1: uniaxial shaking

A uniaxial shaking mechanism illustrated in Figs. 7A and 7B was used as the shaking unit 1, the motor was rotated at 120 rpm, and back-and-forth movement was carried out at a frequency of 2 Hz.

### Shaking unit 2: biaxial shaking

The culture container was shaken by using the biaxial shaking mechanism illustrated in Fig. 8 as the shaking unit 2.

### Information acquisition unit 1 for acquiring information regarding the detachment progressing direction: observation of cell sheet

The observation unit 11 illustrated in Fig. 1 was used as the information acquisition unit 1 to acquire the cell detachment progressing direction.

### Information acquisition unit 2 for acquiring information regarding detachment progressing direction: referencing database

A database stored in the cell detachment system was referenced as the information acquisition unit 2 to acquire the cell detachment progressing direction.

### Changing unit 1: changing tapping direction

A mechanism illustrated in Fig. 9 was used as the changing unit 1 to change the direction of tapping so as to make the shaking direction and the detachment progressing direction substantially parallel to each other.

### Changing unit 2: changing shaking direction

By changing the shaking direction by using the mechanism illustrated in Fig. 9 as the changing unit 2, the shaking direction and the detachment progressing direction were made substantially parallel to each other.

### Changing unit 3: rotating culture container

A mechanism illustrated in Fig. 10 was used as the changing unit 3 to change the orientation of the culture container (rotate) so as to make the shaking direction and the detachment progressing direction substantially parallel to each other.

### Process flow 1: direction not corrected

In the process flow 1, the cell sheet was processed according to the flow illustrated in Fig. 11.

### Process flow 2: direction corrected

In the process flow 2, the cell sheet was processed according to the flow illustrated in Fig. 12.

### Additional vertical vibrations

Ultrasonic vibrations were applied as additional vertical vibrations to the culture container by using the structure illustrated in Fig. 13.

### Evaluation of detachability

For the detachability evaluation, three items, that is, the detachment time, variation in detachment time, and the cell sheet quality, were evaluated. The detachment time can be measured by counting the time from setting the culture container 8 in the cell detachment system and starting driving the system until detachment is complete and the driving is terminated. Since the time required for detachment differs for each cell type, the detachment time was evaluated by determining the ratio of the detachment time of Example to the detachment time of Comparative Example for the same cell type. The detachment time was evaluated on the basis of the following standards.
A: Outstanding (detachment completed in a time 21% or more shorter than that of Comparative Example)
B: Good (detachment completed in a time 11% to 20% shorter than that of Comparative Example)
C: Effective (detachment completed in a time 1% to 10% shorter than that of Comparative Example)
D: Poor (the detachment time was the same as that of Comparative Example)

Variation in detachment time was evaluated by the value obtained by dividing the standard deviation of the detachment times observed by performing cell sheet detachment five times by the average value of the five times. The variation in detachment time was evaluated on the basis of the following standards.
A: Outstanding (standard deviation/average value was less than 0.1)
B: Good (standard deviation/average value was 0.1 or more and less than 0.3)
C: Effective (standard deviation/average value was 0.3 or more and less than 0.5)
D: Poor(standard deviation/average value was 0.5 or more)

The quality of the cell sheet was evaluated by observing the cell sheet after detachment with a phase contrast microscope and checking whether there were holes and tears in the sheet. The cell sheet was determined as having holes when a hole having a diameter of 500 µm or more was observed. The cell sheet was determined as having tears when a tear having a size of 1 mm or more was observed.

The quality of the cell sheet was evaluated on the basis of the following standards.
A: Outstanding (no holes or tears)
B: Good (no tears)
C: Effective (no holes)
D: Poor (holes and tears were present)

### Example 1

C2C12 cells cultured on the culture container 8 were detached by using the detachment start unit 1, the shaking unit 1, the information acquisition unit 1, and the changing unit 1. The detachment time was evaluated, and it took 12 minutes until completion of the detachment. In addition, variation in the detachment time was evaluated, and the standard deviation was 1.33. The quality of the C2C12 cells in a sheet form was evaluated, and there were no holes or tearing or stretched portions after the detachment, but there were some wrinkles.

### Examples 2 to 14 and Comparative Examples 1 to 7

The detachment time, variation in detachment time, and sheet quality were evaluated in accordance with the combinations of the detachment start unit, the shaking unit, the information acquisition unit, the changing unit, the process flow, the additional vertical vibrations, and the cell type illustrated in Table 1. The evaluation results are indicated in Table 2.

**Table 1: Process conditions in Examples 1-14 and Comparative Examples 1 to 7**

| | Cell type | Detachment start unit | Shaking unit | Information acquisition unit | Changing unit | Process flow | Vertical vibrations |
|---|---|---|---|---|---|---|---|
| Example 1 | C2C12 | 1 | 1 | 1 | 1 | 1 | - |
| Example 2 | C2C12 | 2 | 1 | 1 | 1 | 1 | - |
| Example 3 | C2C12 | 3 | 1 | 1 | 1 | 1 | - |
| Example 4 | C2C12 | 1 | 2 | 1 | 2 | 1 | - |
| Example 5 | C2C12 | 1 | 1 | 1 | 3 | 1 | - |
| Example 6 | C2C12 | 1 | 1 | 2 | 1 | 1 | - |
| Example 7 | C2C12 | 1 | 1 | 1 | 1 | 2 | - |
| Example 8 | C2C12 | 1 | 1 | 1 | 1 | 1 | YES |
| Example 9 | A549 | 1 | 1 | 1 | 1 | 1 | - |
| Example 10 | CHO | 1 | 1 | 1 | 1 | 1 | - |
| Example 11 | MDCK | 1 | 1 | 1 | 1 | 1 | - |
| Example 12 | HEK293 | 1 | 1 | 1 | 1 | 1 | - |
| Example 13 | BAEC | 1 | 1 | 1 | 1 | 1 | - |
| Example 14 | MSC | 1 | 1 | 1 | 1 | 1 | - |
| Comparative Example 1 | C2C12 | 1 | 1 | - | - | - | - |
| Comparative Example 2 | A549 | 1 | 1 | - | - | - | - |
| Comparative Example 3 | HUVEC | 1 | 1 | - | - | - | - |
| Comparative Example 4 | MDCK | 1 | 1 | - | - | - | - |
| Comparative Example 5 | HEK293 | 1 | 1 | - | - | - | - |
| Comparative Example 6 | BAEC | 1 | 1 | - | - | - | - |
| Comparative Example 7 | hMSC | 1 | 1 | - | - | - | - |

**Table 2: Evaluation results of Examples 1 to 14 and Comparative Examples 1 to 7**

| | Detachment time | Variation in detachment time | Quality of sheet |
|---|---|---|---|
| Example 1 | B | B | B |
| Example 2 | B | C | B |
| Example 3 | B | B | B |
| Example 4 | A | A | B |
| Example 5 | B | B | B |
| Example 6 | C | C | C |
| Example 7 | A | A | A |
| Example 8 | A | A | A |
| Example 9 | B | B | B |
| Example 10 | B | B | B |
| Example 11 | B | B | B |
| Example 12 | B | B | B |
| Example 13 | B | B | B |
| Example 14 | B | B | B |
| Comparative Example 1 | D | D | D |
| Comparative Example 2 | D | D | D |
| Comparative Example 3 | D | D | D |
| Comparative Example 4 | D | D | D |
| Comparative Example 5 | D | D | D |
| Comparative Example 6 | D | D | D |
| Comparative Example 7 | D | D | D |

According to the present disclosure, a cell detachment system that efficiently detaches a cell sheet while reducing tearing of the cell sheet can be provided. In addition, according to the present disclosure, a cell detachment method that efficiently detaches a cell sheet while reducing tearing of the cell sheet can be provided.

While the present disclosure has been described with reference to exemplary embodiments, it is to be understood that the disclosure is not limited to the disclosed exemplary embodiments. The scope of the following claims is to be accorded the broadest interpretation so as to encompass all such modifications and equivalent structures and functions.

## Claims

1. A cell detachment system that detaches a cell sheet adhering to a culture container from the culture container (8), the cell detachment system comprising:
a detachment start unit that starts detachment of the cell sheet;
an information acquisition unit that acquires information regarding a detachment progressing direction of the cell sheet after start of the detachment of the cell sheet;
a shaking unit (3) that shakes the culture container (8); and
a setting unit that sets, on a basis of the information regarding the detachment progressing direction, the detachment progressing direction and a direction of the shaking to be substantially parallel to each other.

2. The cell detachment system according to Claim 1, wherein the detachment start unit includes a tapping unit that taps the culture container (8).

3. The cell detachment system according to Claim 2, wherein the setting unit sets a direction of the tapping on a basis of the information regarding the detachment progressing direction; and
the tapping unit taps the culture container (8) on a basis of the set direction of the tapping.

4. The cell detachment system according to any one of Claims 1 to 3, wherein the detachment start unit includes a liquid discharging unit that causes a water flow to contact the cell sheet by discharging a liquid.

5. The cell detachment system according to any one of Claims 1 to 4, wherein the setting unit sets the direction of the shaking on a basis of the information regarding the detachment progressing direction; and
the shaking unit (3) shakes the culture container (8) on a basis of the set direction of the shaking.

6. The cell detachment system according to any one of Claims 1 to 5, wherein the setting unit sets the direction of the shaking on a basis of the information regarding the detachment progressing direction; and
the cell detachment system further comprises a changing unit that changes orientation of the culture container (8) on a basis of the set direction of the shaking.

7. The cell detachment system according to any one of Claims 1 to 6, wherein the information acquisition unit acquires observation results of the cell sheet.

8. The cell detachment system according to any one of Claims 1 to 7, wherein the information acquisition unit references a database that contains information regarding the culture container (8) and the information regarding the detachment progressing direction.

9. The cell detachment system according to any one of Claims 1 to 8, wherein the detachment start unit applies vibrations in an ultrasonic band to the culture container (8).

10. The cell detachment system according to any one of Claims 1 to 9, further comprising an ultrasonic wave generating unit that applies vibrations in an ultrasonic band to the culture container (8).

11. A cell detachment method that detaches a cell sheet adhering to a culture container (8) from the culture container (8) by using a cell detachment system, the method comprising:
a detachment start process of starting detachment of the cell sheet;
an information acquisition process of acquiring information regarding a detachment progressing direction of the cell sheet after start of the detachment of the cell sheet;
a shaking process of shaking the culture container (8); and
a setting process of setting, on a basis of the information regarding the detachment progressing direction, the detachment progressing direction and a direction of the shaking to be substantially parallel to each other.
